# EUROPEAN PATENT APPLICATION

(11) **EP 4 609 782 A1**
(43) Date of publication of application: **03.09.2025**
(21) Application number: 22963638.6
(22) Date of filing: 28.12.2022
(51) Int. Cl.: A61B 5/00, A61B 5/055, G06T 7/00, G06T 7/62, G06T 7/11

(54) **METHOD FOR CALCULATING DEMENTIA-RELATED INDEX ON BASIS OF VOLUME OF EXTRACEREBRAL CEREBROSPINAL FLUID, AND ANALYSIS APPARATUS**

(30) Priority: 28.10.2022 KR 20220141232
(71) Applicant: Samsung Life Public Welfare Foundation, Seoul 04348 (KR)
(72) Inventor: SEO, Sang Won, Seoul 06351 (KR); JANG, Hye Min, Seoul 06351 (KR); PARK, Yu Hyun, Seoul 06351 (KR); GU, Yuna, Seoul 06351 (KR)
(74) Representative: Ostertag & Partner Patentanwälte mbB
(86) International application number: PCT/KR2022/021527
(87) International publication number: WO 2024/090677

(57) **Abstract**

This method for calculating a dementia-related index from a brain image comprises steps in which an analysis apparatus: receives a brain image of a subject; identifies a region of interest from the brain image; calculates the volume or the area of the region of interest; and calculates a dementia-related index of the subject on the basis of the volume or the area.

## Description

### Technical Field

The following technology to be described relates to a method of calculating dementia-related information on the basis of a brain image.

### Background Art

Dementia refers to a syndrome causing impairment in cognitive functions such as memory, language, and judgment. Although there are many different types of dementia, Alzheimer's disease is the most common form of dementia.

Dementia is a progressive disease that develops over a long period of time, with pathological changes accumulating before the appearance of clinical symptoms. Thus, early diagnosis of dementia is critical in terms of delaying and managing the onset of dementia symptoms.

Brain images of magnetic resonance imaging (MRI), positron emission tomography (PET), and the like are used to diagnose dementia. Typically, cortical thickness is utilized as a dementia-related index.

### Disclosure

### Technical Problem

However, the measurements of cortical thickness vary depending on the imaging equipment (vendor) used in medical institutions. Thus, cortical thickness has some limitations as an accurate index for diagnosing dementia.

The following technology to be described aims to provide a method of calculating dementia-related information on the basis of different regions of interest extractable from a brain image instead of the cerebral cortex.

### Technical Solution

A methods of calculating a dementia-related index based on the volume of an extracerebral cerebrospinal fluid region A methods of calculating a dementia-related index based on the volume of an extracerebral cerebrospinal fluid region includes the following steps: receiving, by an analysis apparatus, a brain image of a subject; identifying, by the analysis apparatus, a region of interest from the brain image; calculating, by the analysis apparatus, the volume or area of the region of interest; and calculating, by the analysis apparatus, a dementia-related index of the subject on the basis of the volume or area.

In another aspect, a method of calculating a dementia-related index based on the volume of an extracerebral cerebrospinal fluid region includes the following steps: receiving, by an analysis apparatus, a brain image of a subject; identifying, by the analysis apparatus, a region of interest from the brain image; and inputting, by the analysis apparatus, the region of interest into a pre-trained learning model to calculate a dementia-related index of the subject.

An analysis apparatus configured to calculate a dementia-related index includes: an input device configured to receive a brain image of a subject; and a computing device configured to identify a region of interest from the brain image and calculate a dementia-related index on the basis of the region of interest. The region of interest includes an extracerebral cerebrospinal fluid region.

### Advantageous Effects

The following technology to be described provides the degree of brain atrophy on the basis of an extracerebral cerebrospinal fluid region that is identified distinctly from other brain structures in a brain image. Thus, the following technology to be described can provide robust dementia-related information regardless of differences in the characteristics of imaging equipment.

### Description of Drawings

FIG. 1 illustrates an example of estimating cortical thickness in a medical image.
FIG. 2 illustrates an example of a system in which a dementia-related index is calculated by analyzing a brain image.
FIG. 3 illustrates an example of a process in which a dementia-related index is calculated by analyzing a brain image.
FIG. 4 illustrates an example of a process in which a dementia-related index is calculated on the basis of an extracerebral cerebrospinal fluid region and a ventricular region.
FIG. 5 shows evaluation results regarding the relevance of lateral ventricular and extracerebral cerebrospinal fluid regions to dementia assessment.
FIG. 6 shows evaluation results regarding the relevance of extracerebral cerebrospinal fluid subregions to dementia assessment.
FIG. 7 shows performance evaluation results of classifiers constructed on the basis of lateral ventricular and extracerebral cerebrospinal fluid regions.
FIG. 8 shows performance evaluation results of classifiers in which patient information is additionally applied to the model of FIG. 7.
FIG. 9 shows performance evaluation results of classifiers in which patient information is applied to lateral ventricular and extracerebral cerebrospinal fluid subregions.
FIG. 10 illustrates an example of a process in which a dementia-related index is calculated using a learning model.
FIG. 11 illustrates an example of an analysis apparatus configured to calculate a dementia-related index.

### Mode for Invention

While the following technology to be described may be variously modified and have various embodiments, specific embodiments will be illustrated in the accompanying drawings and described in detail. However, this is not intended to limit the following technology to be described to the specific embodiments, and it should be understood that the following technology to be described covers all modifications, equivalents, or substitutions included in the spirit and technical scope thereof.

In addition, although terms such as first, second, A, or B may be used to describe various components, such components are not limited by these terms, and these terms are used merely to distinguish one component from another. For example, without departing from the scope of the following technology to be described, a first component may be referred to as a second component. Similarly, a second component may also be referred to as a first component. The term and/or includes any one of a plurality of related descriptions or combinations thereof.

In the terms used herein, it should be understood that singular expressions include plural expressions unless the context clearly indicates otherwise. In addition, it should be understood that the terms such as "including," "comprising," and the like are intended to specify the presence of stated features, integers, steps, operations, components, parts, or combinations thereof but do not preclude the possibility of the presence or addition of one or more other features, integers, steps, operations, components, parts, or combinations thereof.

Before discussing detailed descriptions of the drawings, it is to be clearly noted that the classification of components described herein is merely based on the primary functions of the respective components. In other words, two or more components to be described below may be integrated into a single component, or a single component may be divided into two or more subcomponents depending on the respective functions thereof. Furthermore, it should also be noted that each of the components to be described below may further perform, in addition to the primary function thereof, a part or all of the functions of other components, and other components may be in charge of and perform a part of the primary functions of each component.

Moreover, in performing methods or operation methods, each of the steps constituting such methods may be carried out in an order that differs from what is stated, unless the context clearly specifies any particular order. In other words, each process may be carried out in the exact order stated, performed practically simultaneously, or performed in reverse order.

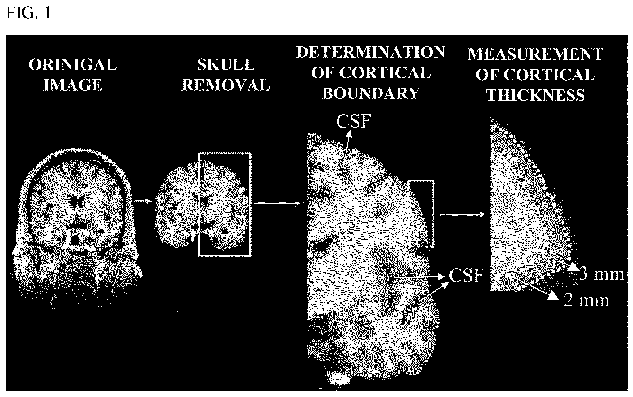

Alzheimer's disease is diagnosed on the basis of cortical thickness as identified in a medical image. FIG. 1 illustrates an example of estimating cortical thickness in a medical image. FIG. 1 illustrates an example of measuring or estimating cortical thickness in an MRI image. In FIG. 1, the cerebral cortex corresponds to the gray region between the solid white and dotted lines. Therefore, cortical thickness is accurately measurable only when the white region inside the solid white line and the gray region between the solid white and dotted lines are clearly distinguished. In the meantime, medical imaging equipment is provided by various vendors, and imaging parameters may vary depending on the equipment type, even among equipment manufactured by the same vendor.

The researchers collected data from the population that visited the affiliated medical institution (Samsung Seoul Hospital). The population includes 605 participants in a normal control group and 616 participants in a dementia group, those who were examined in Samsung Seoul Hospital between 2015 and 2021. All participants underwent dementia assessments, including brain MRI and amyloid PET. In this case, the normal control group consisted of amyloid-negative subjects, and the dementia group consisted of amyloid-positive subjects.

**[Table 1]**

| | | | | | | | |
|---|---|---|---|---|---|---|---|
| Measurement subject | | **Aβ(-) NC** | | | **Aβ(+) ADD** | | |

| Equipment type | | Achieva | Ingenia | P value | Achieva | Ingenia | P value |
|---|---|---|---|---|---|---|---|
| Population size | N | 581 | 24 | | 588 | 28 | |
| Cortical thickness | Frontal | 3.16 ± | 3.17 ± 0.09 | 0.530 | 3.01 ± 0.15 | 3.00 ± 0.12 | 0.762 |
| | Parietal | 3.08 ± 0.11 | 3.08 ± 0.13 | 0.895 | 2.87 ± 0.20 | 2.86 ± 0.17 | 0.747 |
| | **Temporal** | **3.29 ± 0.11** | **3.38 ± 0.13** | **0.001** | 3.06 ± 0.18 | 3.06 ± 0.18 | 0.857 |
| | **Occipital** | **2.99 ± 0.16** | **3.08 ± 0.19** | **0.011** | 2.79 ± 0.19 | 2.78 ± 0.18 | 0.632 |
| | Global | 3.13 ± | 3.17 ± 0.12 | 0.059 | 2.94 ± 0.15 | 2.93 ± 0.14 | 0.711 |

Table 1 shows the measurement results of cortical thickness of the subjects using Archieva and Ingenia, the imaging equipment from the same manufacturer (Philips) used in the medical institution to which the researchers belong. Aβ(-) NC refers to a normal control group without amyloid beta accumulation, and Aβ(+) ADD refers to an Alzheimer's disease patient group with amyloid beta accumulation. In Aβ(+) ADD, the cortical thickness obtained by MRI scan in the Achieva and Ingenia devices showed no significant difference. However, in Aβ(-) NC, the measurements obtained from the temporal and occipital lobes using the two devices showed significant differences. In other words, as the researchers expected, the measured cortical thickness was somewhat different when the medical devices were manufactured by the same vendor but differed in type. This is because there are differences in parameter values set for different imaging equipment.

FIG. 1 shows some regions of an extracerebral cerebrospinal fluid (extracerebral CSF) region. The extracerebral CSF region corresponds to a sulcus region between gyri. The extracerebral CSF region looks black or extremely dark in the MRI image. In other words, the extracerebral CSF region corresponds to a region that is visually distinctly distinguished from the gray region of the cerebral cortex on MRI. Therefore, it can be presumed that the extracerebral CSF region will be a region capable of being relatively accurately identified by segmentation models or image processing techniques of computer devices.

**[Table 2]**

| Measurement subject | | **Aβ(-) NC** | | | **Aβ(+) ADD** | | |
|---|---|---|---|---|---|---|---|
| Equipment type | | Achieva | Ingenia | P value | Achieva | Ingenia | P value |
| Population size | N | 581 | 24 | | 588 | 28 | |
| CSF space | Lateral ventricle | 0.01 ± 0.01 | 0.01 ± 0.00 | 0.378 | 0.02 ± 0.01 | 0.02 ± 0.01 | 0.705 |
| | Extracerebral CSF | 0.46 ± 0.05 | 0.46 ± 0.05 | 0.742 | 0.52 ± 0.05 | 0.51 ± 0.04 | 0.285 |

Table 2 shows measurement results of the length (or size) of the extracerebral CSF region for the population described in Table 1, using the Achieva and Ingenia devices. From a detailed look at the results in Table 2, the length of the extracerebral CSF region was found to show no significant difference between the two devices in both Aβ(+) ADD and Aβ(-) NC. In other words, as the researchers predicted, it can be presumed that the extracerebral CSF region has distinct imaging features compared to the cerebral cortical region. Cortical thickness is an index for the degree of brain atrophy. The more severe the degree of brain atrophy, the smaller the cortical thickness. In addition, the size of the extracerebral CSF region correlates with the degree of brain atrophy. The more severe the degree of brain atrophy, the larger the size of the extracerebral CSF region.

Hereinafter, dementia refers to Alzheimer's dementia.

The following technology to be described is a technology for diagnosing dementia or predicting the possibility of developing dementia on the basis of the extracerebral CSF region.

The size of the extracerebral CSF region may be assessed as the gap or distance between gyri. Alternatively, the size of the extracerebral CSF region may be assessed as the area of a specific sulcus. The size of the area may be calculated from a two-dimensional (2D) image. Alternatively, the size of the extracerebral CSF region may be assessed as the volume of a specific sulcus. The volume may be calculated from a three-dimensional (3D) image or 2D slices.

Hereinafter, an apparatus configured to calculate a dementia-related index of a subject by analyzing a brain image is referred to as an analysis apparatus. The analysis apparatus may have forms of a computer device, such as a personal computer (PC), a smart device, a network server, a chipset dedicated to data processing, and the like.

The analysis apparatus may calculate the size or volume of a specific extracerebral CSF region in a brain image using a conventional image processing technique. Alternatively, the analysis apparatus may calculate the size or volume of a specific extracerebral CSF region using a deep learning network-type model. The analysis apparatus may calculate the degree of brain atrophy on the basis of the size or volume of the extracerebral CSF region. Furthermore, the analysis apparatus may produce a diagnosis or prediction results of dementia for the subject by analyzing a brain image.

The analysis apparatus calculates a dementia-related index by analyzing a brain image. In this case, the dementia-related index corresponds to an index or information related to dementia progression. The dementia-related index may include at least one piece of information including the size or volume of the extracerebral CSF region, the degree of brain atrophy assessed by the size or volume of the extracerebral CSF region, and the presence or absence of dementia (or the degree of dementia progression) assessed by the size/volume (or the degree of brain atrophy)of the extracerebral CSF region.

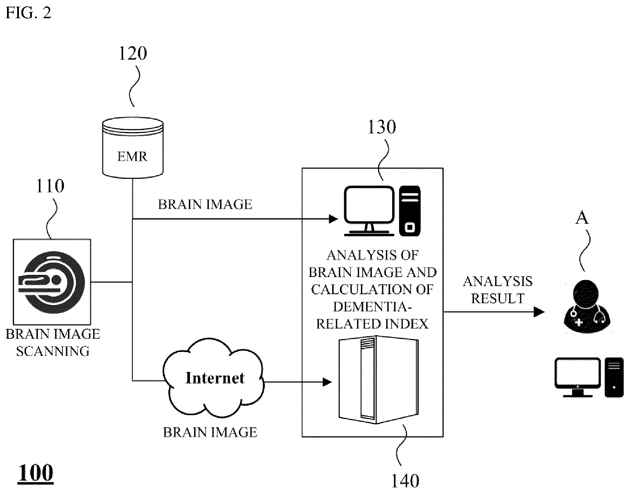

FIG. 2 illustrates an example of a system 100 in which a dementia-related index is calculated by analyzing a brain image. In FIG. 2, a computer terminal 130 and a server 140 are illustrated as examples of the analysis apparatus.

Medical imaging equipment 110 produces a brain image (for example, an MRI image) of a patient. The brain image produced by the medical imaging equipment 110 may be stored in a separate database such as an electronic medical record (EMR) 120.

In FIG. 2, a user A may analyze the brain image using the computer terminal 130 to obtain the dementia-related index. The computer terminal 130 may receive a brain image of a specific subject from the medical imaging equipment 110 or the EMR 120 via a wired or wireless network. In some cases, the computer terminal 130 may be an apparatus physically connected to the medical imaging equipment 110. The computer terminal 130 extracts an extracerebral CSF region from the brain image. The computer terminal 130 may calculate the dementia-related index on the basis of the extracerebral CSF region. (i) The computer terminal 130 may estimate the size or volume of the extracerebral CSF region. (ii) The computer terminal 130 may estimate the degree of brain atrophy depending on the size or volume of the extracerebral CSF region. (iii) The computer terminal 130 may estimate the presence or absence of dementia, the possibility of developing dementia, the degree of dementia progression, or the like, depending on the size/ volume of the extracerebral CSF region or the degree of brain atrophy. The user A may confirm the analysis results on the computer terminal 130.

The server 140 may receive a brain image of a specific subject from the medical imaging equipment 110 or the EMR 120. The server 140 extracts an extracerebral CSF region from the brain image. The server 140 may calculate the dementia-related index on the basis of the extracerebral CSF region. (i) The server 140 may estimate the size or volume of the extracerebral CSF region. (ii) The server 140 may estimate the degree of brain atrophy depending on the size or volume of the extracerebral CSF region. (iii) The server 140 may estimate the presence or absence of dementia, the possibility of developing dementia, the degree of dementia progression, or the like, depending on the size/ volume of the extracerebral CSF region or the degree of brain atrophy. The server 140 may transmit the analysis results of the brain image to a terminal of the user A. The user A may confirm the analysis results through the user terminal.

The computer terminal 130 and/or the server 140 may also store the analysis results in the EMR 120.

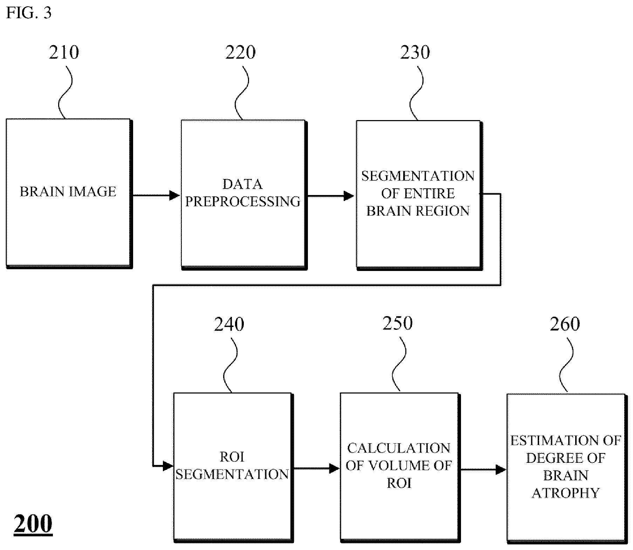

FIG. 3 illustrates an example of a process 200 in which a dementia-related index is calculated by analyzing a brain image.

The analysis apparatus receives a brain image (MRI image) of a subject (210).

The analysis apparatus may consistently preprocess data of the received brain image (220). Preprocessing the data may be a process of extracting surface structures or models of brain structures from the received image to produce a mask. For example, the analysis apparatus may extract the entire brain region from a brain MRI image using the CIVET pipeline. The analysis apparatus may extract brain regions from MRI slices. The analysis apparatus may extract brain regions from continuous MRI slices to extract 3D brain regions. To sum up, (i) the analysis apparatus may produce a mask of the entire brain region from the brain image. In addition, (ii) the analysis apparatus may also produce a mask of a specific region from the brain image. The specific region may include at least one of an extracerebral CSF region, extracerebral CSF subregions, a ventricular region, and ventricular subregions. The extracerebral CSF subregions and the ventricular subregions are to be described later.

In the meantime, the data preprocessing process for mask production may be an optional process. Among image processing techniques, when using a technique to automatically extract a region of interest (ROI), the analysis apparatus may not provide the mask in advance.

The analysis apparatus may segment the entire brain region from the brain image of the subject (230). The analysis apparatus may segment the entire brain region using the mask provided during the data preprocessing process. Alternatively, the analysis apparatus may segment the entire brain region using a segmentation model.

The analysis apparatus may segment the ROI from the entire brain region (240). The analysis apparatus may segment the ROI using the mask provided during the data preprocessing process. Alternatively, the analysis apparatus may segment the ROI using a segmentation model. The ROI includes the extracerebral CSF region. The ROI may include other regions in addition to the extracerebral CSF region. In addition, the ROI may be composed of subregions capable of being extracted from the extracerebral CSF region. Specific ROI is to be described later.

The analysis apparatus may calculate the volume of the segmented ROI (250). The analysis apparatus may calculate the volume of the 3D ROI using commonly used programs or algorithms. In the meantime, the analysis apparatus may calculate the area of the 2D ROI from an MRI slice. The analysis apparatus may calculate the area of the ROI from all slices or a specific slice selected.

The analysis apparatus may estimate the degree of brain atrophy on the basis of the volume or area of the ROI (260). The dementia-related index in FIG. 3 is the degree of brain atrophy. The volume of the ROI or the area of a specific point (or points) has a consistent correlation with the degree of brain atrophy. The correlation between the volume (or area) of the ROI and the degree of brain atrophy may be provided in advance in a table form. In this case, the analysis apparatus may estimate the degree of brain atrophy of the subject on the basis of the calculated volume (or area) of the ROI. Alternatively, the analysis apparatus may estimate the degree of brain atrophy of the subject using a function with the calculated volume (or area) of the ROI as a variable. This function may be a known formula. Alternatively, this function may be a formula provided in advance through regression analysis.

The ROI includes the extracerebral CSF region. Furthermore, the ROI may include at least some of the individual subregions of the extracerebral CSF region. In addition, the ROI may also include a ventricular region that is neither white nor gray on MRI and thus is relatively distinctly distinguished. Moreover, the ROI may include at least some of the individual subregions of the ventricular region. The ROI may be one of several regions or any one of several possible combinations thereof. ROI candidates are as listed in Table 3 below.

**[Table 3]**

| Entire region | Subregions |
|---|---|
| 1. Extracerebral CSF region | 1-1. Frontal region |
| | 1-2. Temporal region |
| | 1-3. Parietal region |
| | 1-4. Occipital region |
| 2. Ventricular region | 2-1. Lateral ventricle |
| | 2-2. Third ventricle |
| | 2-3. Fourth ventricle |

Possible ROIs are as follows. (i) The ROI may be at least one of the entire extracerebral CSF region and the entire ventricular region. (ii) In addition, the ROI may be at least one of the entire extracerebral CSF region and the ventricular subregions. (iii) In addition, the ROI may be at least one of the extracerebral CSF subregions and the entire ventricular region. (iv) In addition, the ROI may be at least one of the extracerebral CSF subregions and the ventricular subregions.

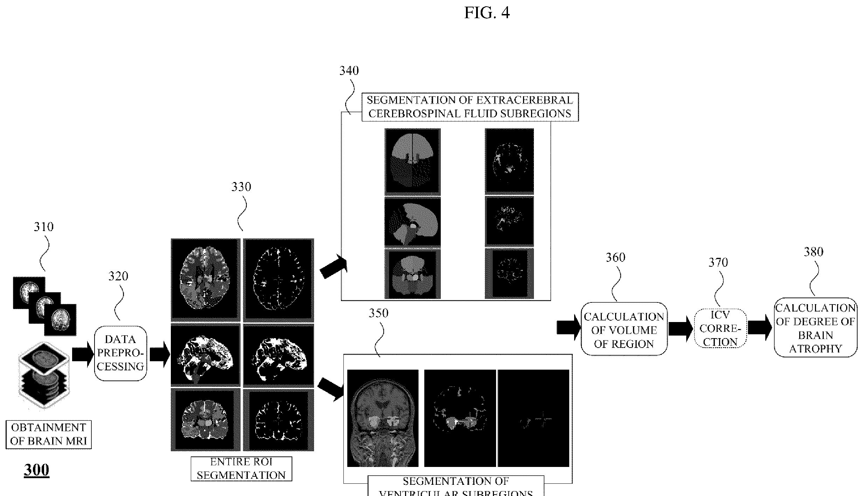

FIG. 4 illustrates an example of a process 300 in which a dementia-related index is calculated on the basis of an extracerebral CSF region and a ventricular region. FIG. 4 illustrates an example of calculating the dementia-related index using the subregions among ROIs.

The analysis apparatus receives a brain image (MRI image) of a subject (310).

The analysis apparatus may consistently preprocess data of the received brain image

(320). Preprocessing the data may be a process of extracting surface structures or models of brain structures or models from the received image to produce a mask. The analysis apparatus may produce a mask of the entire brain region from the brain image. In addition, the analysis apparatus may also produce a mask of a specific region from the brain image. The specific region may include at least one of the extracerebral CSF region, the extracerebral CSF subregions, the ventricular region, and the ventricular subregions.

In the meantime, the data preprocessing process for mask production may be an optional process. Among image processing techniques, when using a technique to automatically extract the ROI, the analysis apparatus may not provide the mask in advance.

The analysis apparatus may segment the entire ROI region from the brain image of the subject (330). The analysis apparatus may segment the entire brain region using the mask provided during the data preprocessing process. Alternatively, the analysis apparatus may segment the entire brain region using a segmentation model.

The analysis apparatus may segment a specific ROI from the entire brain region. The analysis apparatus may segment at least one of the extracerebral CSF subregions using the mask (340). In addition, the analysis apparatus may segment at least one of the ventricular subregions using the mask (350). The analysis apparatus may segment a target ROI using a segmentation model.

The analysis apparatus may calculate the volume of the segmented ROI (360). The analysis apparatus may calculate the volume of the 3D ROI using commonly used programs or algorithms. In the meantime, the analysis apparatus may calculate the area of the 2D ROI from an MRI slice. The analysis apparatus may calculate the area of the ROI from all slices or a specific slice selected.

The analysis apparatus may normalize each brain region using the intracranial volume (ICV) of the subject (370). The analysis apparatus may consistently correct the size or volume of the target ROI on the basis of the ICV. The ICV-based correction process may be an optional process.

The analysis apparatus may estimate the degree of brain atrophy on the basis of the volume or area of the ROI (380). The analysis apparatus may estimate the degree of brain atrophy on the basis of the volume or area corrected on the basis of the ICV (380). For example, the analysis apparatus may normalize the volume by dividing the volume (or area) of the ROI by the ICV.

The dementia-related index in FIG. 4 is the degree of brain atrophy. The analysis apparatus may estimate the degree of brain atrophy of the subject by comparing the calculated volume (or area) of the ROI and a reference provided in advance. Alternatively, the analysis apparatus may estimate the degree of brain atrophy of the subject using a function with the calculated volume (or area) of the ROI as a variable.

The researchers selected the ROI for calculating the dementia-related index.

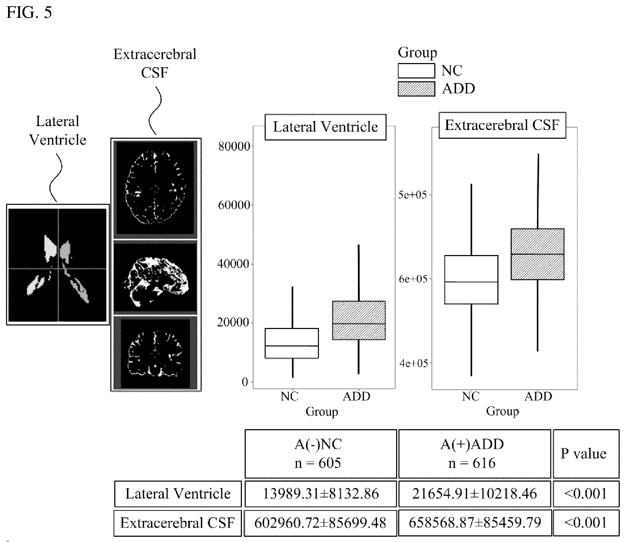

The researchers identified the relevance of the lateral ventricles of the ventricular subregions and the entire extracerebral CSF region to dementia. FIG. 5 shows evaluation results regarding the relevance of the lateral ventricular and extracerebral CSF regions to dementia assessment. In FIG. 5, A(-) NC refers to a normal control group without amyloid beta accumulation, and A(+) ADD refers to an Alzheimer's disease patient group with amyloid beta accumulation. From a detailed look at the results in FIG. 5, each of the lateral ventricular and extracerebral CSF regions may be the ROI. (i) The lateral ventricular region may be an index enabling the normal control group and the patient group to be distinguished (p < 0.001). In addition, (ii) the entire extracerebral CSF region may also be an index enabling the normal control group and the patient group to be distinguished (p < 0.001).

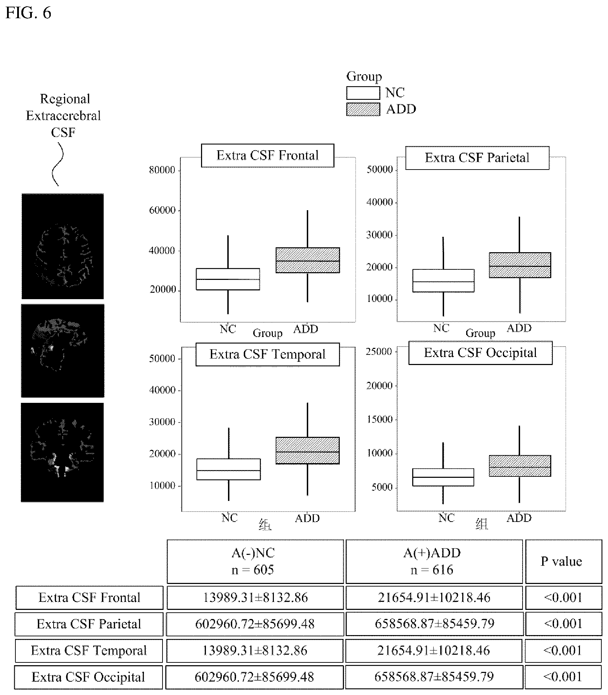

The researchers identified the relevance of each of the extracerebral CSF subregions to dementia. FIG. 6 shows evaluation results regarding the relevance of the extracerebral CSF subregions to dementia assessment. The extracerebral CSF subregions include the frontal region (F), the temporal region (T), the parietal region (P), and the occipital region (O). In FIG. 6, A(-) NC refers to a normal control group without amyloid beta accumulation, and A(+) ADD refers to an Alzheimer's disease patient group with amyloid beta accumulation. From a detailed look at the results in FIG. 6, each of the extracerebral CSF subregions may be the ROI (p < 0.001)

The researchers constructed a model (classifier) to calculate the dementia-related index on the basis of the selected ROI. The classifier was implemented as a machine learning model. The researcher used 70% of the data from the population described above as learning data and the remainder of 30% as verification data. The researchers performed logistic regression using glm() in R. However, the classifier may also be implemented as other models, such as deep learning models.

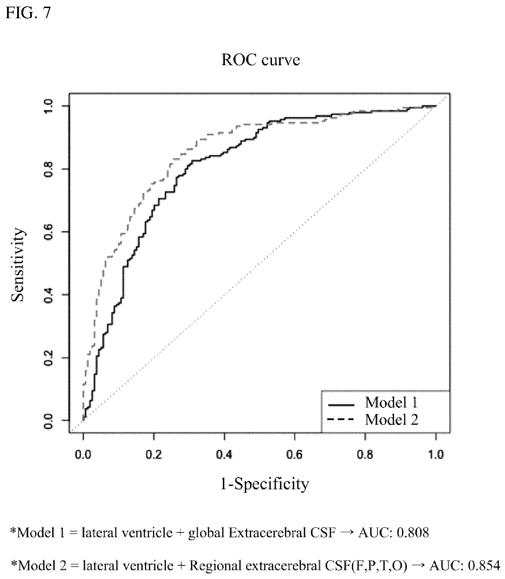

FIG. 7 shows performance evaluation results of classifiers constructed on the basis of the lateral ventricular and extracerebral CSF regions. FIG. 7 shows the performance of two models (Model 1 and Model 2). Model 1 and Model 2 are models to calculate the dementia-related index using only brain images. Model 1 is a model that uses the lateral ventricular and entire extracerebral CSF regions as the ROIs. Model is a model that uses the lateral ventricular and extracerebral CSF subregions as the ROIs. The area under the receiver operating characteristic (ROC) curve (AUC) of Model 1 was 0.808. The subregions of the extracerebral CSF region used in Model 2 are the frontal region (F), the temporal region (T), the parietal region (P), and the occipital region (O). The AUC of Model was 0.854. Although Model 2 slightly outperformed Model 1, both Model 1 and Model 2 were found to be sufficiently meaningful in diagnosing or predicting dementia.

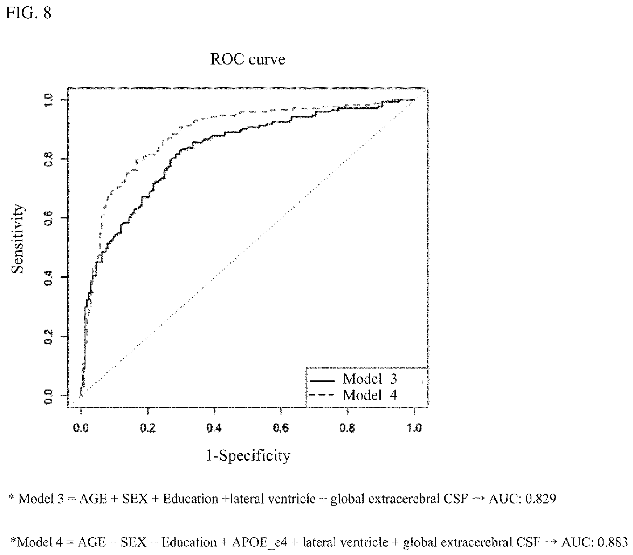

FIG. 8 shows performance evaluation results of classifiers in which patient information is additionally applied to the lateral ventricular and entire extracerebral CSF regions. FIG. 8 shows the performance of two models (Model 3 and Model 4). Model 3 and Model 4 are models to calculate the dementia-related index using brain images and patient information. Model 3 and Model 4 are pre-trained models using brain images and patient information.

Model 3 is a model in which additional patient information (age, gender, and education level) is applied in addition to Model 1. In other words, Model 3 is a model that calculates the dementia-related index using (i) the lateral ventricular and entire extracerebral CSF regions extracted from the brain image and (ii) the patient information (age, gender, and education level). The AUC of Model 3 was 0.829.

Model 4 is a model in which additional patient information (age, gender, and education level) and clinical information (APOE e4) are applied in addition to Model 1. In other words, Model 3 is a model that calculates the dementia-related index using (i) the lateral ventricular and entire extracerebral CSF regions extracted from the brain image, (ii) the patient information (age, gender, and education level), and (iii) the patient clinical information (APOE e4). APOE e4 refers to the genotype information of a dementia-related gene. The AUC of Model 4 was 0.883.

Both Model 3 and Model 4 outperformed Model 1. Therefore, both Model 3 and Model 4 are found to be sufficiently meaningful in diagnosing or predicting dementia.

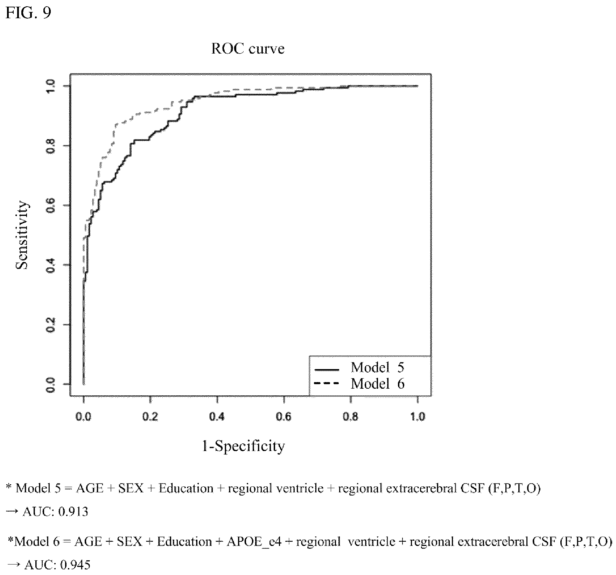

FIG. 9 shows performance evaluation results of classifiers in which patient information is applied to the lateral ventricular and extracerebral CSF subregions. FIG. 9 shows the performance of two models (Model 5 and Model 6). Model 5 and Model 6 are models to calculate the dementia-related index using brain images and patient information. Model 5 and Model 6 are pre-trained models using brain images and patient information.

Model 5 is a model that calculates the dementia-related index using (i) the ventricular subregions and the extracerebral CSF subregions (F, P, T, and O) extracted from the brain image and (ii) the patient information (age, gender, and education level). The AUC of Model 5 was 0.889.

Model 6 is a model that calculates the dementia-related index using (i) the ventricular subregions and the extracerebral CSF subregions (F, P, T, and O) extracted from the brain image, (ii) the patient information (age, gender, and education level), and (iii) the patient clinical information (APOE e4). The AUC of Model 6 was 0.932.

Both Model 5 and Model 6 outperformed Model 2. Therefore, both Model 5 and Model 6 are found to be sufficiently meaningful in diagnosing or predicting dementia.

To sum up, it is found that (i) the entire extracerebral CSF region, (ii) all the subregions of the extracerebral CSF region, (iii) some regions of the subregions of the extracerebral CSF region, (iv) the lateral ventricles + the entire extracerebral CSF region, (v) the lateral ventricles + all the subregions of the extracerebral CSF region, (vi) the lateral ventricles + some regions of the subregions of the extracerebral CSF region, and (vii) the ventricular subregions + at least some regions of the subregions of the extracerebral CSF region are all meaningful as the ROIs associated with the dementia-related index. In the meantime, the classifiers experimentally using the subregions of the extracerebral CSF region as the ROIs slightly outperformed the classifiers using the entire extracerebral CSF region. In addition, the classifiers using only brain MRI also showed sufficiently significant performance. Furthermore, when applying additional information, such as patient information, in addition to brain MRI images, the performance of the classifiers was confirmed to be improved.

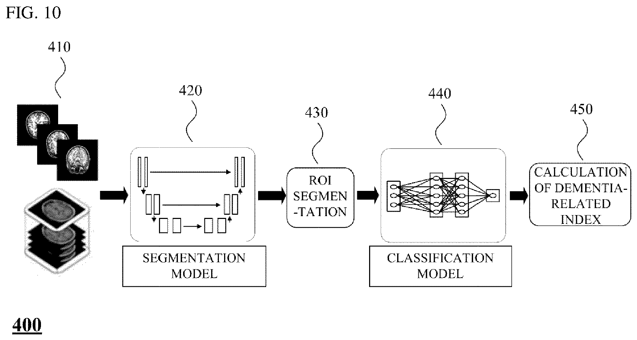

FIG. 10 illustrates an example of a process 400 in which a dementia-related index is calculated using a learning model. FIG. 10 shows a case where the learning model is used in both the process of extracting an ROI from a brain MRI image and the process of predicting a dementia-related index on the basis of the ROI.

The analysis apparatus receives a brain image (MRI image) of a subject (410).

The analysis apparatus may input the received brain image into a pre-trained segmentation model (420). The segmentation model may be implemented as models of various types or structures. For example, the segmentation model may be a model based on U-net. The segmentation model may segment various ROIs depending on the types or learning processes thereof. The segmentation model may segment the ROI from 3D brain images. Alternatively, the segmentation model may segment the ROI from individual 2D slices. As described above, some regions of various regions may be used as the ROI. The segmentation model may be provided in advance for each specific ROI. For example, the segmentation model may be diverse as follows: (i) a model that segments the entire extracerebral CSF region, (ii) a model that segments at least some of the extracerebral CSF subregions, (iii) a model that segments the entire extracerebral CSF region + the lateral ventricular region, (iv) a model that segments at least some of the extracerebral CSF subregions + the lateral ventricular region, and the like. The segmentation model may be constructed in advance as a model that segments any one of the various ROIs depending on the learning data and learning processes.

The analysis apparatus may obtain the results (ROI identification) output by the segmentation model (430).

The analysis apparatus inputs the ROI into a pre-trained classification model (440). The classification model is a model that calculates the dementia-related index by inputting images or images and patient information.

The classification model is a machine learning model. Therefore, the classification model may be any one of various types of models. For example, the classification model may be a model implemented using any one of the following methods: decision trees, random forests (RFs), K-nearest neighbors (KNN), Naive Bayes, support vector machines (SVMs), artificial neural networks (ANNs), regression models, and the like. There are also various types of ANN models. For example, the classification model may be a convolutional neural network (CNN)-based model.

The analysis apparatus may obtain the dementia-related index output by the classification model (450). The dementia-related index may be information such as the volume of the ROI, the degree of brain atrophy, or the degree of dementia progression.

The classification model may calculate the dementia-related index using only the ROI. In addition, the classification model may calculate the dementia-related index by inputting the ROI and patient information (age, gender, education level, and the like) into the classification model. Furthermore, the classification model may also calculate the dementia-related index by inputting the ROI, patient information (age, gender, education level, and the like), and clinical information (APOE e4 and the like) into the classification model.

In the meantime, unlike FIG. 10, the learning model may be used in any one of the processes of extracting the ROI or predicting the dementia-related index. In other words, (i) the analysis apparatus may extract the ROI using the segmentation model and estimate the degree of brain atrophy by calculating the volume of the extracted ROI. Calculating the volume and determining the degree of brain atrophy are as described in FIG. 3 or FIG. 4. Alternatively, (ii) the analysis apparatus may extract the ROI using a mask and calculate the dementia-related index by inputting the extracted ROI into the classification model in FIG. 10. The process of extracting the ROI using the mask is as described in FIG. 3 or FIG. 4.

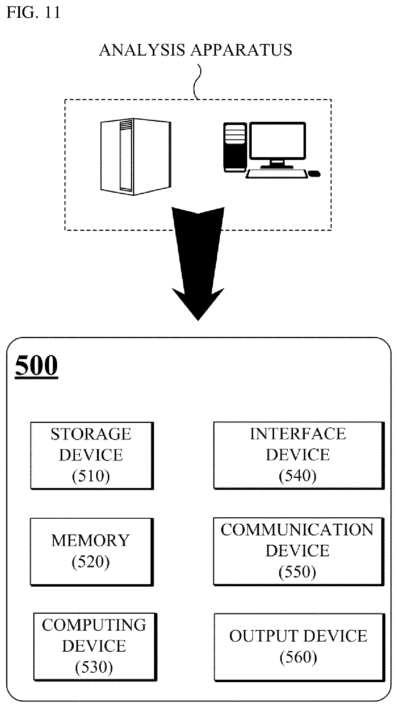

FIG. 11 illustrates an example of an analysis apparatus 500 configured to calculate a dementia-related index. The analysis apparatus 500 corresponds to the analysis apparatus (130 and 140 in FIG. 1) described above. The analysis apparatus 500 may be physically implemented in various forms. For example, the analysis apparatus 500 may have forms of a computer device, such as a PC, a network server, a chipset dedicated to data processing, and the like.

The analysis apparatus 500 may include a storage device 510, a memory 520, a computing device 530, an interface device 540, a communication device 550, and an output device 560.

The storage device 510 may store a brain image (MRI image), which is produced in medical imaging equipment, of a subject.

The storage device 510 may store patient information and clinical information about the subject. The patient information and the clinical information are as described above.

The storage device 510 may store a code or program that calculates the dementia-related index from the brain image.

The storage device 510 may store a mask (or masks) extracted from the brain image.

The storage device 510 may store a segmentation model for extracting an ROI from the brain image. The segmentation model is a pre-trained model.

The storage device 510 may store a classification model that calculates the dementia-related index by receiving the ROI. The classification model is a pre-trained model.

The storage device 510 may store the dementia-related index of the subject.

The memory 520 may store data, information, and the like produced during a process in which the analysis apparatus 500 calculates the dementia-related index from the brain image.

The interface device 540 is a device configured to receive certain commands and data from outside. The interface device 540 may receive the brain image of the subject from a physically connected input device or an external storage device. The interface device 540 may receive patient information and/or clinical information of the subject from a physically connected input device or an external storage device. The interface device 540 may also deliver the dementia-related index calculated on the basis of the brain image to an external object.

The communication device 550 refers to a configuration that receives and transmits certain information via a wired or wireless network. The communication device 550 may receive the brain image of the subject from an external object. The communication device 550 may receive patient information and/or clinical information of the subject from an external object. The communication device 550 may also transmit the dementia-related index calculated on the basis of the brain image to an external object such as a user terminal.

The interface device 540 and the communication device 550 are configurations that exchange certain data from a user or other physical objects and thus may also be comprehensively referred to as input/output devices. In terms of information or data input functions, the interface device 540 and the communication device 550 may also be referred to as input devices.

The output device 560 is a device configured to output certain information. The output device 560 may output an interface required during a data processing process, the brain image, the ROI segmented from the brain image, the dementia-related index calculated on the basis of the ROI, and the like.

The computing device 530 may consistently preprocess the brain image of the subject. The data preprocessing process is as described in FIG. 3. The computing device 530 may produce the mask (or masks) for segmenting the entire brain region and the ROI through the data preprocessing process.

The computing device 530 may segment the entire brain region from the brain image using the mask for the entire brain region. Furthermore, the computing device 530 may segment a target ROI from the entire brain region using the mask for a specific ROI. As described above, the ROI may be diverse. For example, the ROI may be any one of the entire extracerebral CSF region, the extracerebral CSF subregions, the entire extracerebral CSF region + the lateral ventricular region, and the extracerebral CSF subregions + the lateral ventricular region.

The computing device 530 may segment the ROI from the received brain image using the segmentation model.

The computing device 530 may calculate the volume or area of the segmented ROI. The computing device 530 may calculate the volume or area of the ROI using any one of the commonly used programs for calculating the volume of the brain region. Furthermore, the computing device 530 may normalize the initially calculated volume of the ROI on the basis of the ICV.

The computing device 530 may estimate the degree of brain atrophy on the basis of the volume or area of the final ROI. Alternatively, the computing device 530 may estimate the degree of dementia of the subject on the basis of the volume or area of the final ROI. Alternatively, the computing device 530 may estimate the degree of dementia of the subject on the basis of the degree of brain atrophy of the subject.

The computing device 530 may calculate the dementia-related index by inputting the ROI into the classification model constructed in advance. In addition, the computing device 530 may calculate the dementia-related index by inputting the ROI and patient information (age, gender, education level, and the like) into the classification model. Furthermore, the computing device 530 may calculate the dementia-related index by inputting the ROI, patient information (age, gender, education level, and the like), and clinical information (APOE e4 and the like) into the classification model.

The computing device 530 may be a device, such as a processor, an application processor (AP), or a program-embedded chip, that processes data and processes certain operations.

In addition, a method of processing the brain image described above, calculating the dementia-related index, or predicting dementia may be implemented using a program (or application) incorporating an executable algorithm that can be run on a computer. This program may be stored in a transitory or non-transitory computer-readable medium and provided.

The non-transitory computer-readable medium refers to a medium that semi-permanently stores data and is readable by a device, other than a medium, such as a register, cache, or memory, that stores data for a short period of time. Specifically, various applications or programs described above may be stored in non-transitory computer-readable media such as a compact disc (CD), a digital versatile disc (DVD), a hard disk, a Blu-ray disc, a Universal Serial Bus (USB), a memory card, a read-only memory (ROM), a programmable ROM (PROM), erasable PROM (EPROM), electrically EPROM (EEPROM), or a flash memory and provided.

The transitory computer-readable medium refers to various types of random-access memory (RAM) such as static RAM (SRAM), dynamic RAM (DRAM), synchronous DRAM (SDRAM), double data rate SDRAM (DDR SDRAM), enhanced SDRAM (ESDRAM), Synclink DRAM (SLDRAM), and Direct Rambus RAM (DRRAM).

The embodiments and the accompanying drawings described herein merely clearly illustrate some of the technical ideas included in the technology described above. In addition, it is apparent that all variations and specific embodiments that can be easily derived by those skilled in the art within the scope of the technical idea included in this specification and drawings of the above-described technology fall within the scope of the appended claims of the above-described technology.

## Claims

1. A method of calculating a dementia-related index based on a volume of an extracerebral cerebrospinal fluid region, the method comprising:
receiving, by an analysis apparatus, a brain image of a subject;
identifying, by the analysis apparatus, a region of interest from the brain image;
calculating, by the analysis apparatus, a volume or area of the region of interest; and
calculating, by the analysis apparatus, a dementia-related index of the subject on the basis of the volume or area,
wherein the region of interest comprises an extracerebral cerebrospinal fluid region.

2. The method of claim 1, wherein the dementia-related index comprises at least one of degree of brain atrophy, presence or absence of dementia, and degree of dementia progression.

3. The method of claim 1, wherein the analysis apparatus identifies the region of interest using a mask produced by preprocessing the brain image.

4. The method of claim 1, wherein the region of interest further comprises a ventricular region, and
the ventricular region comprises lateral ventricles.

5. The method of claim 1, wherein the region of interest is a subregion comprised in the extracerebral cerebrospinal fluid region, and
the subregion comprises at least one of a frontal region, a temporal region, a parietal region, and an occipital region.

6. A method of calculating a dementia-related index based on a volume of an extracerebral cerebrospinal fluid region, the method comprising:
receiving, by an analysis apparatus, a brain image of a subject;
identifying, by the analysis apparatus, a region of interest from the brain image; and
inputting, by the analysis apparatus, the region of interest into a pre-trained learning model to calculate a dementia-related index of the subject,
wherein the region of interest comprises an extracerebral cerebrospinal fluid region.

7. The method of claim 6, wherein the analysis apparatus identifies the region of interest by inputting the brain image into a pre-trained segmentation model.

8. The method of claim 6, wherein the region of interest comprises the entire extracerebral cerebrospinal fluid region and a lateral ventricular region.

9. The method of claim 6, wherein the region of interest is a subregion comprised in the extracerebral cerebrospinal fluid region, and
the subregion comprises at least one of a frontal region, a temporal region, a parietal region, and an occipital region.

10. The method of claim 9, wherein the region of interest further comprises a lateral ventricular region.

11. The method of claim 9, wherein the analysis apparatus further inputs additional information of the subject to the learning model to calculate the dementia-related index, and
the additional information comprises at least one of age, gender, education level, and APOE e4 genotype of the subject.

12. An analysis apparatus configured to calculate a dementia-related index, the analysis apparatus comprising:
an input device configured to receive a brain image of a subject; and
a computing device configured to identify a region of interest from the brain image and calculate a dementia-related index on the basis of the region of interest,
wherein the region of interest comprises an extracerebral cerebrospinal fluid region.

13. The analysis apparatus of claim 12, wherein the computing device identifies the region of interest using a mask produced by preprocessing the brain image.

14. The analysis apparatus of claim 12, wherein the computing device identifies the region of interest by inputting the brain image into a pre-trained segmentation model.

15. The analysis apparatus of claim 12, wherein the computing device calculates the dementia-related index on the basis of a volume or area of the region of interest.

16. The analysis apparatus of claim 12, wherein the computing device calculates the dementia-related index by inputting the region of interest into a pre-trained learning model.

17. The analysis apparatus of claim 12, wherein the region of interest comprises the entire extracerebral cerebrospinal fluid region and a lateral ventricular region.

18. The analysis apparatus of claim 12, wherein the region of interest is a subregion comprised in the extracerebral cerebrospinal fluid region, and
the subregion comprises at least one of a frontal region, a temporal region, a parietal region, and an occipital region.

19. The analysis apparatus of claim 12, wherein the region of interest is a subregion comprised in the extracerebral cerebrospinal fluid region, and
the subregion comprises a lateral ventricular region and at least one of a frontal region, a temporal region, a parietal region, and an occipital region.
